(19) Europäisches Patentamt
European Patent Office
Office européen des brevets

(11) Publication number: **0 017 074**
**A1**

(12) # EUROPEAN PATENT APPLICATION

(21) Application number: 80101416.8

(22) Date of filing: 18.03.80

(51) Int. Cl.[3]: C 07 D 221/26
C 07 D 221/22, C 07 D 453/06

(30) Priority: 26.03.79 US 23613

(43) Date of publication of application:
15.10.80 Bulletin 80/21

(84) Designated Contracting States:
BE CH DE FR GB IT NL SE

(71) Applicant: STERLING DRUG INC.
90 Park Avenue
New York New York(US)

(72) Inventor: Michne, William Francis
R.D.2, Averill Park
New York(US)

(72) Inventor: Lewis, Thomas Richard
9 North Street
Delmar, New York(US)

(74) Representative: Baillie, Iain Cameron et al,
c/o Ladas & Parry Blumenstrasse 48
D-8000 München 2(DE)

(54) **Process and intermediates for preparing 11-(alkoxy- and alkylmercapto-3-oxoalkyl)-2,6-methano-3-benzazocines.**

(57)

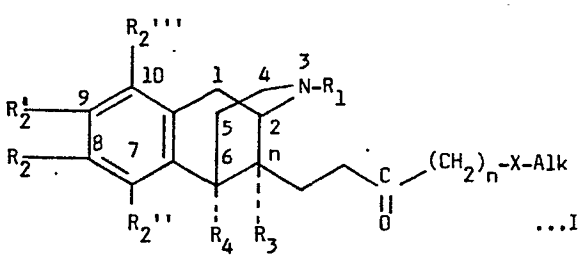

...I

where $R_1$ is hydrogen, lower-alkyl, cyclo-alkyl-lower-alkyl, phenyl-lower-alkyl, lower-alkenyl or lower-alkynyl; three of $R_2$, $R_2'$, $R_2''$ and $R_2'''$ are hydrogen and the fourth is hydroxy; $R_3$ and $R_4$ are each hydrogen or lower-alkyl, or $R_3$ and $R_4$ together are divalent lower-alkylene, $-(CH_2)_m-$, where m is one of the integers 3 and 4; n is one of the integers 2-4; X is oxygen or sulfur (-O- or -S-); and Alk is lower-alkyl, which comprises heating in an acid medium, a compound having the formula:

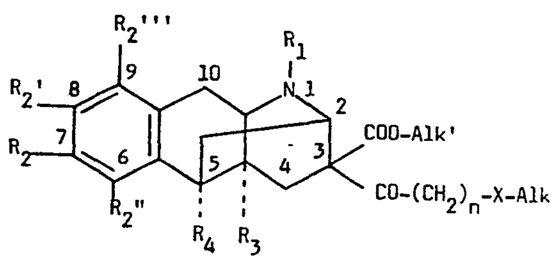

where three of $R_2$, $R_2'$, $R_2''$ and $R_2'''$ are hydrogen and the fourth is methoxymethoxy; $R_1$, $R_3$, $R_4$, n, X and Alk have the meanings given above, and Alk' is lower-alkyl.

EP 0 017 074 A1

"PROCESS AND INTERMEDIATES FOR PREPARING 11-(ALKOXY-AND ALKYLMERCAPTO-3-OXOALKYL)-2,6-METHANO-3-BENZAZOCINES"

# D E S C R I P T I O N

This invention relates to a novel process for preparing 3-$R_1$-7-, 8-, 9- or 10-hydroxy-11(ax)-$R_3$-6(eq)-$R_4$-11-(eq)-[$CH_2CH_2CO(CH_2)_n$-X-Alk]-1,2,3,4,5,6-hexahydro-2,6-methano-3-benzazocines having the formula:

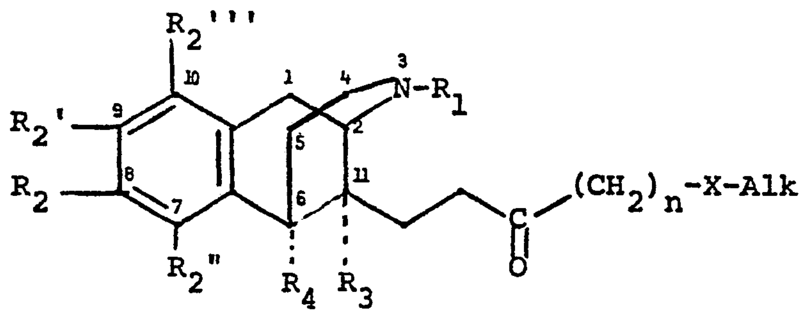

...I

where $R_1$ is hydrogen, lower-alkyl, cycloalkyl-lower-alkyl, phenyl-lower-alkyl, lower-alkenyl or lower-alkynyl; three of $R_2$, $R_2'$, $R_2''$ and $R_2'''$ are hydrogen and the fourth is hydroxy; $R_3$ and $R_4$ are each hydrogen or lower-alkyl, or $R_3$ and $R_4$ together are divalent lower-alkylene, $-(CH_2)_m$, where m is one of the integers 3 and 4; n is one of the integers 2-4; X is oxygen or sulfur (-O- or -S-); and Alk is lower-alkyl, and is an improvement in the process described in Belgian Patent 864,950.

As used herein, the term lower-alkyl means saturated, acyclic groups which may be straight or branched containing from one to about seven carbon atoms as exemplified by methyl, ethyl, propyl, isopropyl, butyl, hexyl or heptyl.

As used herein, the terms lower-alkenyl and lower-alkynyl mean monovalent groups of from three to seven carbon atoms containing one double or triple bond as illustrated for example, by 2-propenyl, 2-butenyl, 4-pentenyl, 3-methyl-2—butenyl, 1—methyl—2—propenyl, 2—methyl—2—propenyl,

2-propynyl, 2-butynyl, 4-pentynyl, 2-hexynyl and the like.

As used herein, the term cycloalkyl means saturated carbocyclic groups containing from three to seven ring carbon atoms as illustrated, for example, by cyclopropyl, cyclobutyl, cyclopentyl, cyclohexyl, 2-methylcyclobutyl, 4-ethylcyclohexyl and the like.

According to the process of the present invention, the compounds of Formula I are prepared by heating, in an acid medium, for example with formic acid either alone or in an organic solvent, for example toluene, xylene or mesitylene, at a temperature from 100-150°C., or with a benzyl-di-lower-alkylammonium formate or a tri-lower-alkylammonium formate at a temperature from 120-150°C., a lower-alkyl 1-$R_1$-3-[Alk-X$(CH_2)_n$-CO]-4aα-$R_3$-5α-$R_4$-6-, 7-, 8- or 9-methoxymethoxy—1,2,3,4,4a,5,10,10a-octahydro-2,5-methanobenzo[g]quinoline-3—carboxylate having the formula:

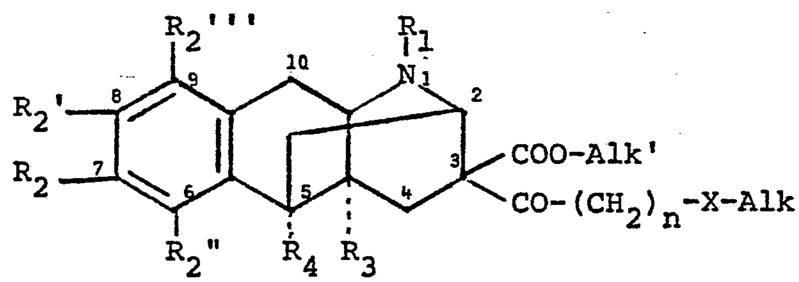

...II

where three of $R_2$, $R_2'$, $R_2''$ and $R_2'''$ are hydrogen and the fourth is methoxymethoxy; $R_1$, $R_3$, $R_4$, n, X and Alk have the meanings given above; and Alk' is lower-alkyl.

Under the acid conditions present during the heating of the compounds of Formula II in formic acid or formate solution, the compounds of Formula I where one of $R_2$, $R_2'$, $R_2''$ and $R_2'''$ is hydroxy are produced from the compounds of Formula II where the corresponding group is methoxymethoxy ($CH_3OCH_2O$) by simultaneous ring opening of the compounds of Formula II between the 2- and 3-carbon atoms, decarbalkoxylation of the resulting 2,6-methano-3-benzazocine moiety and cleavage of the methoxymethoxy group.

The above-described process for the preparation of the compounds of Formula I via the compounds of Formula

II represents a substantial improvement over the known process for preparing the compounds of Formula I described in Belgian Patent 864,950, because the present process combines in one step, i.e. the ring opening/decarbalkoxylation and simultaneous ether cleavage, the conversion of the compounds of Formula II to the compounds of Formula I, whereas according to the known process, compounds corresponding to Formula II where three of $R_2$, $R_2'$, $R_2''$, and $R_2'''$ are hydrogen and the fourth is lower-alkoxy are heated in an acid medium to effect ring opening/decarbalkoxylation to the corresponding ethers corresponding to Formula I which are then cleaved in a separate step to the corresponding hydroxy compounds (i.e. the compounds where three of $R_2$, $R_2'$, $R_2''$ and $R_2'''$ are hydrogen and the fourth is hydroxy). The starting compounds of Formula II wherein one of the indicated radicals is methoxymethoxy are readily obtained by the process described above from the corresponding hydroxy compound. The related lower-alkoxy-substituted starting materials employed in the prior art reaction are obtained from the corresponding hydrcxy-substituted compound by the Williamson synthesis, which process is no more efficient and probably less efficient than the process for obtaining the methoxymethoxy compound.

The compounds of Formula II where one of $R_2$, $R_2'$, $R_2''$ and $R_2'''$ is methoxymethoxy are prepared by reaction of the corresponding compounds where the subject group is hydroxy with dimethoxymethane in the presence of a catalytic amount of a strong acid in an inert organic solvent. The reaction is carried out by refluxing a solution of the reactants in the chosen solvent, for example chloroform, methylene dichloride, ethylene dichloride and the like, under a Soxhlet extractor containing molecular sieves having a pore size sufficient to trap and hold molecules of methanol. In this way the methanol produced in the reversible reaction is removed from the reaction mixture as it is formed, and the reaction proceeds to completion. It has been found that 4A molecular sieves have a porosity of the proper size for

this purpose.

The compounds of Formula II where one of $R_2$, $R_2'$, $R_2''$ and $R_2'''$ is methoxymethoxy are particularly useful as intermediates for preparing the compounds of Formula I where the subject group is hydroxy and which contain acid sensitive groups elsewhere in the molecule, for example the compounds of Formula I where $R_1$ is cycloalkyl-lower-alkyl where cycloalkyl is cyclopropyl, since as indicated above the methoxymethoxy group is readily cleaved under mild acid conditions.

The compounds of Formula II are prepared according to the procedure described in U.S. Patent 4,119,628 which comprises reacting a lower-alkyl 1-$R_1$-4aα-$R_3$-5α-$R_4$-6-, 7-, 8- or 9-methoxymethoxy-1,2,3,4,4a,5,10,10a-octahydro-2,5-methanobenzo-[g]quinoline-3-carboxylate having the formula:

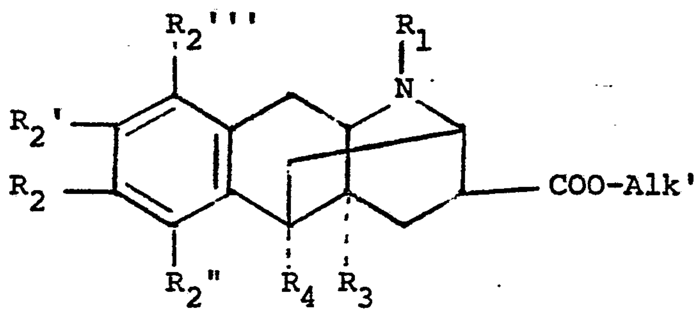

...III

where three of $R_2$, $R_2'$, $R_2''$ and $R_2'''$ are hydrogen and the fourth is methoxymethoxy; and $R_1$, $R_3$, $R_4$ and Alk' have the meanings given above with an alkali metal amide, and reacting the resulting alkali metal salt with an appropriate acid halide, Alk-X-$(CH_2)_n$-CO-Hal, where Alk, X and n have the meanings given above, and Hal is halogen.

The compounds of Formula III and the method for their preparation are disclosed in U.S. Patent 4,100,164.

Due to the presence of a basic amino grouping, the free base form represented by Formula I above reacts with organic and inorganic acids to form acid-addition salts. The acid-addition salt forms are prepared from any organic or inorganic acid. They are obtained in conventional fashion, for instance either by direct mixing of the base with

the acid or, when this is not appropiate, by dissolving either or both of the base and the acid separately in water or an organic solvent and mixing the two solutions, or by dissolving both the base and the acid together in a solvent. The resulting acid-addition salt is isolated by filtration, if it is insoluble in the reaction medium, or by evaporation of the reaction medium to leave the acid-addition salt as a residue. The acid moieties or anions in these salt forms are in themselves neither novel nor critical and therefore can be any acid anion or acid-like substance capable of salt formation with the base.

Representative acids for the formation of the acid-addition salts include formic acid, acetic acid, isobutyric acid, alpha-mercaptopropionic acid, trifluoroacetic acid, malic acid, fumaric acid, succinic acid, succinamic acid, tannic acid, glutamic acid, tartaric acid, oxalic acid, pyromucic acid, citric acid, lactic acid, glycolic acid, gluconic acid, saccharic acid, ascorbic acid, penicillin, benzoic acid, phthalic acid, salicylic acid, 3,5-dinitrobenzoic acid, anthranilic acid, cholic acid, 2-pyridinecarboxylic acid, pamoic acid, 3-hydroxy-2-naphthoic acid, picric acid, quinic acid, tropic acid, 3-indoleacetic acid, barbituric acid, sulfamic acid, methanesulfonic acid, ethanesulfonic acid, isethionic acid, benzenesulfonic acid, p-toluenesulfonic acid, butylarsonic acid, methanephosphonic acid, acidic resins, hydrofluoric acid, hydrochloric acid, hydrobromic acid, hydriodic acid, perchloric acid, nitric acid, sulfuric acid, phosphoric acid, arsenic acid, and the like.

The compounds of Formula I can exist in enantiomeric forms separable into enantiomers. If desired, the isolation or the production of a particular enantiomeric form can be accomplished by application of general principles known in the prior art. In the nomenclature employed for the compounds of Formula I herein, "ax" stands for axial and "eq" for equatorial, and the configurations are given with reference to the hydroaromatic ring. Thus, the

6(eq), 11(ax) substituents of Formula I are in the cis configuration, whereas the 6(eq), 11(eq) substituents are in the trans configuration.

In the nomenclature employed for the compounds of Formulas II and III, again configurations are given with reference to the hydroaromatic ring, and the designation "β" indicates the cis configuration relative to the 2,5-methano bridge of the compounds of Formulas II and III. Conversely, the designation "α" indicates the trans configuration relative to the same group.

In standard pharmacological test procedures, the compounds of Formula I and the acid-addition salts thereof have been found useful as depressants of the central nervous system, and more particularly have been found useful as analgesics and as antagonists of strong analgesics such as phenazocine, meperidine and morphine.

The compounds of Formula I can be administered in the same manner as known analgesics and antagonists of strong analgesics, i.e. parenterally or orally in any of the conventional pharmaceutical forms, as for instance solutions, suspensions, tablets, capsules, and the like.

The useful properties of the compounds of Formula I were demonstrated by standard pharmacological procedures readily carried out by technicians having ordinary skill in pharmacological test procedures, so that the actual determination of numerical biological data definitive for a particular test compound can be ascertained without the need for any extensive experimentation.

The test procedures used to determine analgesic and analgesic antagonist activities of the compounds of Formula I have been described in detail in the prior art and are as follows: The acetylcholine-induced abdominal constriction test, which is a primary analgesic screening test designed to measure the ability of a test agent to suppress acetylcholine-induced abdominal constriction in mice, described by Collier et al., Brit. J. Pharmacol. Chemotherap. 32, 295 (1968); a modification of the anti-bradykinin test, which is also

a primary analgesic screening procedure, described by Berkowitz et al., J. Pharmacol. Exptl. Therap. 177, 500-508 (1971), Blane et al., J. Pharm. Pharmacol. 19, 367-373 (1967), Botha et al., Eur. J. Pharmacol. 6, 312-321 (1969) and Deffenu et al., J. Pharm. Pharmacol. 18, 135 (1966); the phenyl-p-quinone-induced writhing test, also a primary analgesic screening test, designed to measure the ability of a test agent to prevent phenyl-p-quinone-induced writhing in mice, described by Pearl and Harris, J. Pharmacol. Exptl. Therap. 154, 319-323 (1966); the rat tail flick radiant thermal heat analgesic (agonist) test described by D'Amour and Smith, J. Pharmacol. Exptl. Therap. 72, 74 (1941) as modified by Bass and VanderBrook, J. Am. Pharm. Assoc., Sci. Ed. 41, 569 (1956); and the narcotic antagonist test using phenazocine, meperidine or morphine, which is designed to measure the ability of a test agent to antagonize the effect of phenazocine, meperidine or morphine in the above-indicated rat tail flick response test, described by Harris and Pierson, J. Pharmacol. Exptl. Therap. 143, 141 (1964).

The structures of the compounds of Formula I were established by the mode of synthesis, by elementary analyses and by mass, infrared and nuclear mangetic resonance spectra. The course of reactions and homogeneity of the products were ascertained by thin layer chromatography.

The manner of carrying out the process of the invention will now be described so as to enable any person skilled in the art to which it pertains to use the same. The melting points are uncorrected.

Example 1

A solution of 2.0 g. (0.0062 mole) of methyl 7-hydroxy-1,4aα,5α-trimethyl-1,2,3,4,4a,5,10,10a-octahydro-2,5-methanobenzo[g]quinoline-3-carboxylate and 0.75 g. (0.0068 mole) of ethanesulfonic acid in 75 ml. of dichloromethane and 10 ml. of dimethoxymethane was heated under reflux under a Soxhlet extractor containing 4A molecular sieves for one hundred and twenty hours, and then cooled and poured into an ice/dilute sodium hydroxide solution. The mixture

was extracted three times with methylene dichloride, and the extracts were dried over anhydrous magnesium sulfate and taken to dryness to give 2.2 g. of an oily gum which solidified to give material having m.p. 63-73°C. Recrystalization of the crude material from methanol afforded two crops, 0.86 g., m.p. 79-81°C., and 0.69 g., m.p. 72-79°C. of methyl 7-methoxymethoxy-1,4aα,5α-trimethyl-1,2,-3,4,4a,5,10,10a-octahydro-2,5-methanobenzo[g]quinoline-3-carboxylate.

The latter, dissolved in anhydrous tetrahydrofuran, is added to a slight molor excess of lithium diisopropylamide (prepared by reaction of butyl lithium in hexane with diisopropylamine) in tetrahydrofuran at -70°C. under a nitrogen atmosphere with stirring. When addition of the ester to the lithium diisopropylamide is complete, the mixture is stirred at -70°C. for one hour, treated with a solution containing one molar equivalent of γ-methylmercaptobutyryl chloride in anhydrous tetrahydrofuran, and the mixture stirred for about one hour at -70°C.

The reaction mixture is then poured into saturated sodium bicarbonate, the solution extracted with methylene dichloride, the extracts dried over anhydrous magnesium sulfate and taken to dryness to give methyl 7-methoxymethoxy-1,4aα,5α-trimethyl-3-(1-oxo-4-methylmercaptobutyl)-1,2,3,4,4a,5,10,10a-octahydro-2,5-methanobenzo[g]quinoline-3-carboxylate.

The latter, without purification, is dissolved in five volumes of trimethylammonium formate (prepared by reaction of two molar equivalents of trimethylamine with five molar equivalents of formic acid), and the solution is heated under reflux while following the course of the reaction by thin layer chromatography. When reaction is complete (in fifteen minutes to a half hour), the excess trimethylammonium formate is removed *in vacuo*. The residue is suspended in aqueous ammonium bicarbonate, extracted with methylene dichloride, and the organic extracts, after drying, are taken to dryness to give the product in

the form of the free base. The latter is dissolved in acetone, the solution is treated with a molar equivalent amount of ethanesulfonic acid, and the solution is diluted with diethyl ether. The solid which separates is collected and dried to give 3,6(eq),-11(ax)-trimethyl-8-hydroxy-11(eq)-(6-methylmercapto-3-oxohexyl)-1,2,3,4,5,6,-hexahydro-2,6-methano-3-benzazocine ethanesulfonate, m.p. 175.5-180°C.

Anal. Calcd. for $C_{22}H_{33}NO_2S.C_2H_5SO_3H$: C, 59.35; H, 8.06; N, 2.88.
Found: C, 59.34; H, 8.23; N, 2.78.

Example 2

Following a procedure similar to that described in Example 1 above, methyl 7-methoxymethoxy-1,4aα,5α-trimethyl-1,2,3,4,4a,5,10,10a-octahydro-2,5-methanobenzo[g]-quinoline-3-carboxylate, dissolved in anhydrous tetrahydrofuran, is added to a slight molar excess of lithium diisopropylamide in tetrahydrofuran, the resulting lithium salt is reacted with a solution containing a slight molar excess of γ-propylmercaptobutyryl chloride in tetrahydrofuran, and the product is worked up in an alkaline medium as before to give methyl 7-methoxymethoxy-1,4aα,5α-trimethyl-3-(1-oxo-4-propylmercaptobutyl)-1,2,3,4,4a,5,10,10a-octahydro-2,5-methanobenzo[g]quinoline-3-carboxylate.

The latter, without purification, is refluxed in about five volumes of trimethylammonium formate, and the product is isolated as before and converted to the sulfate salt. Recrystallization of the latter from ethanol/diethyl ether gives 3,6(eq),11(ax)-trimethyl-8-hydroxy-11(eq)-(6-propylmercapto-3-oxohexyl)-1,2,3,4,5,6-hexahydro-2,6-methano-3-benzazocine sulfate, m.p. 198-202°C.

Anal. Calcd. for $C_{24}H_{37}NO_2S.H_2SO_4$: C, 57.45; H, 7.80; N, 2.79.
Found: C, 57.44; H, 7.87; N, 2.78.

Example 3

Following a procedure similar to that described in Example 1 above, methyl 7-methoxymethoxy-1,4aα,5α-trimethyl-1,2,3,4,4a,5,10,10a-octahydro-2,5-methanobenzo[g]-quinoline-3-carboxylate, dissolved in tetrahydrofuran, is reacted with a slight molar excess of lithium diisopropylamide in tetrahydrofuran, the resulting lithium salt is reacted with a slight molar excess of γ-methoxybutyryl chloride dissolved in tetrahydrofuran, and the product is worked-up in an alkaline medium as before to give methyl 7-methoxymethoxy-1,4aα,5α-trimethyl-3-(1-oxo-4-methoxybutyl)-1,2,3,-4,4a,5,10,10a-octahydro-2,5-methanobenzo[g]quinoline-3-carboxylate.

The latter, without purification, is heated under reflux in approximately five volumes of trimethylammonium formate, and the product isolated as before and converted to the methanesulfonate salt. Recrystallization of the latter from acetone gives 3,6(eq),11(ax)-trimethyl-8-hydroxy-11(eq)-(6-methoxy-3-oxohexyl)-1,2,3,4,5,6-hexahydro-2,6-methano-3-benzazocine methanesulfonate, m.p. 211-214°C.

Anal. Calcd. for $C_{22}H_{33}NO_3.CH_3SO_3H$: C, 60.63; H, 8.19; N, 3.07.
Found: C, 60.83; H, 8.37; N, 3.07.

BIOLOGICAL TEST RESULTS

The compounds of Formula I are generally active in the acetylcholine-induced abdominal constriction test, a primary analgesic screening test, the anti-bradykinin test and also in the rat tail flick radiant thermal heat antagonist test.

Thus the $ED_{50}$'s in the acetylcholine-induced abdominal constriction test and the anti-bradykinin test and the $AD_{50}$'s vs. phenazocine and morphine in the narcotic antagonist test of the compound of Example 1 were found to be, respectively, 0.1-1.0 mg./kg. (s.c.), 0.071 mg./kg. (s.c.), 0.0034 mg./kg. (s.c.) and 0.18 mg./kg. (s.c.). The same compound was found to be inactive in the tail flick agonist test at 120 mg./kg. (s.c.).

The compound of Example 2 was found to be essentially inactive in the actylcholine-induced abdominal constriction test (60% inhibition/20 mg./kg.; 53%/10 mg./kg.; 40%/1 mg./kg.; and 7%/0.1 mg./kg. - all s.c.) and also in the anti-bradykinin test [0/5 protected at 1.0 and 10.0 mg./kg. (s.c.)]. The $AD_{50}$ vs. phenazocine in the tail flick antagonist test was found to be 0.046 mg./kg. (s.c.).

The $ED_{50}$ of the compound of Example 3 in the acetylcholine-induced abdominal constriction test was found to be 0.20 mg./kg. (s.c.), and the $AD_{50}$ vs. phenazocine in the narcotic antagonist test was found to be 17 mg./kg. (s.c.). This species was found to be inactive in the tail flick agonist test at 120 mg./kg. (s.c.).

CLAIMS

1. A process for preparing a compound having the Formula I (herein), where $R_1$ is hydrogen, lower-alkyl, cycloalkyl-lower-alkyl, phenyl-lower-alkyl, lower-alkenyl or lower-alkynyl; three of $R_2$, $R_2'$, $R_2''$ and $R_2'''$ are hydrogen and the fourth is hydroxy; $R_3$ and $R_4$ are each hydrogen or lower-alkyl, or $R_3$ and $R_4$ together are divalent lower-alkylene, $-(CH_2)_m-$, where m is one of the integers 3 and 4; n is one of the integers 2-4; X is oxygen or sulfur (-O- or -S-); and Alk is lower-alkyl, which comprises heating, in an acid medium, a compound having the Formula II (herein), where three of $R_2$, $R_2'$, $R_2''$ and $R_2'''$ are hydrogen and the fourth is methoxymethoxy; $R_1$, $R_3$, $R_4$, n, X and Alk have the meanings given above, and Alk' is lower-alkyl.

2. A process according to claim 1, where the acid medium is either formic acid alone or formic acid dissolved in an organic solvent; a benzyl-di-lower-alkylammonium formate; or a tri-lower-alkylammonium formate.

3. A process according to claim 2, where the reaction is carried out in a formic acid/mesitylene medium, preferably at a temperature from 100-150°C.

4. A process according to claim 2, where the reaction is carried out in the presence of a tri-lower-alkylammonium formate, preferably at a temperature from 120-150°C.

5. A process according to any of claims 1 to 4, for preparing 3,6(eq),11(ax)-trimethyl-8-hydroxy-11(eq)-(6-methylmercapto-3-oxohexyl)-1,2,3,4,5,6-hexahydro-2,6-methano-3-benzazocine from a lower-alkyl 7-methoxymethoxy-1,4aα,5α-trimethyl-3-(1-oxo-4-methylmercaptobutyl)-1,2,3,4,4a,5,10,10a-octahydro-2,5-methanobenzo[g]quinoline-3-carboxylate.

6. A process according to any of claims 1 to 4, for preparing 3,6(eq),11(ax)-trimethyl-8-hydroxy-11(eq)-(6-propylmercapto-3-oxohexyl)-1,2,3,4,5,6-hexahydro-2,6-methano-3-benzazocine from a lower-alkyl 7-methoxymethoxy-1,4aα,5α-trimethyl-3-(1-oxo-4-propylmercaptobutyl)-1,2,3,4,4a,5,10,10a-octahydro-2,5-methanobenzo[g]quinoline-3-carboxylate.

7. A process according to any of claims 1 to 4, for

preparing 3,6(eq),11(ax)-trimethyl-8-hydroxy-11(eq)-(6-methoxy-3-oxohexyl)-1,2,3,4,5,6-hexahydro-2,6-methano-3-benzazocine from a lower-alkyl 7-methoxymethoxy-1,4aα,5α-trimethyl-3-(1-oxo-4-methoxybutyl)-1,2,3,4,4a,5,10,10a-octahydro-2,5-methanobenzo[g]quinoline-3-carboxylate.

8. A compound having the Formula II (herein), where $R_1$ is hydrogen, lower-alkyl, cycloalkyl-lower-alkyl, phenyl-lower-alkyl, lower-alkenyl or lower-alkynyl; three of $R_2$, $R_2'$, $R_2''$ and $R_2'''$ are hydrogen and the fourth is methoxymethoxy; $R_3$ and $R_4$ are each hydrogen or lower-alkyl, or $R_3$ and $R_4$ together are divalent lower-alkylene, $-(CH_2)_m-$, where m is one of the integers 3 and 4; n is one of the integers 2-4; X is oxygen or sulfur (-O- or -S-); and Alk and Alk' are each lower-alkyl.

9. A compound according to claim 8, where each of $R_1$, $R_3$, $R_4$, Alk and Alk' are lower-alkyl; each of $R_2'$, $R_2''$ and $R_2'''$ are hydrogen; and n is the integer 3.

0017074

European Patent Office

# EUROPEAN SEARCH REPORT

Application number
EP 80 10 1416

## DOCUMENTS CONSIDERED TO BE RELEVANT

| Category | Citation of document with indication, where appropriate, of relevant passages | Relevant to claim |
|---|---|---|
| | GB - A - 1 514 571 (STERLING DRUG INC.)<br>* Claim 30; page 25 *<br>-- | 1 |
| D | BE - A - 864 950 (STERLING DRUG INC.)<br>* Pages 2-3 *<br>-- | 1 |

CLASSIFICATION OF THE APPLICATION (Int. Cl. 3)

C 07 D 221/26
221/22
C 07 D 453/06

TECHNICAL FIELDS SEARCHED (Int.Cl. 3)

C 07 D 221/26
221/22

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 11.07.1980 | BRIGHENTI |